# EUROPEAN PATENT SPECIFICATION

(11) **EP 1 854 494 B1**
(45) Date of publication and mention of the grant of the patent: **27.06.2012**
(21) Application number: 07108079.0
(22) Date of filing: 11.05.2007
(51) Int. Cl.: A61M 16/20, F16K 15/14, A61M 16/06

(54) **Improvements Relating to Respiratory Masks**
Verbesserungen im Zusammenhang mit Atemmasken
Améliorations associées aux masques respiratoires

(30) Priority: 12.05.2006 GB 0609400
(43) Date of publication of application: 14.11.2007
(73) Proprietor: Intersurgical AG, Vaduz (LI)
(72) Inventor: Bowsher, Richard Francis, Reading, Berkshire RG41 7DB (GB)
(74) Representative: Adamson Jones

(56) References cited:
- US-A- 4 811 730
- US-A- 5 465 712
- US-A- 6 039 042

## Description

This invention relates to respiratory masks, and in particular to respiratory masks including valve assemblies.

Patients suffering from conditions such as hypoxemia, severe trauma, and acute myocardial infarction, are typically provided with supplemental oxygen for inhalation. A widely-used method for delivering supplemental oxygen to a patient is to use a respiratory mask.

Simple respiratory masks generally fit over a patient's nose and mouth, and are secured around the patient's head by an elastic strap. Oxygen is delivered through a small-bore tube connected to the base of the mask, and openings on each side of the mask enable the escape of exhaled gases and also serve as atmospheric air entrainment ports. The Fraction of Inspired Oxygen (FIO₂) varies with the patient's inspiratory flow rate and respiratory pattern, as well as mask fit.

In order to deliver a higher concentration of oxygen to a patient, high concentration respiratory masks including a reservoir bag are typically used. In particular, high concentration respiratory masks generally include a reservoir bag that is charged with oxygen during use, so that sufficient oxygen is available to meet peak inspiratory flow demands of the patient.

High concentration oxygen masks are generally of two different types: non-rebreathing masks and partial rebreathing masks. Non-rebreathing masks have a number of one-way valves that ensure an oxygen supply with minimal dilution from the entrainment of exhaled gases and/or atmospheric air. In particular, an inhalation valve between the reservoir bag and the mask body enables oxygen to be inhaled from the reservoir bag, but prevents entry of expired gas into the reservoir bag, and one or more exhalation valves in the wall of the mask body enable expired carbon dioxide to leave the mask, but limit the inhalation of atmospheric air. Partial rebreathing masks permit mixing of exhaled gases with the oxygen supply so as to conserve oxygen, and may or may not include inhalation and/or exhalation valves.

High concentration respiratory masks are generally used to deliver concentrations in the range of FlO₂≥ 0.40. In practice, FlO₂ is limited because most conventional respiratory masks dilute the inspired oxygen with entrained atmospheric air because of poor fit of the mask to the face. A tight fit is therefore necessary to deliver a high FlO₂.

Inhalation and exhalation valves therefore act to increase oxygen delivery and reduce rebreathing of exhaled gases in high concentration respiratory masks. Conventional inhalation and exhalation valves for respiratory masks generally comprise a deformable silicone washer that is mounted about a central retainer and occludes one or more apertures in a rest configuration. A sufficient differential between the pressure within the respiratory mask, and either the oxygen supply pressure or the surrounding atmospheric pressure, causes the silicone washer to deform away from the one or more apertures and hence allow the passage of gas through the valve.

However, conventional respiratory masks including such valves suffer from numerous disadvantages. In particular, the multi-component construction of the valves, and also the need for separate inhalation and exhalation valves, leads to increased manufacturing costs relative to simple respiratory masks. Furthermore, the central retainer of conventional inhalation and exhalation valves limits the size of the apertures allowing the passage of gas through the valves, and hence causes undesirable resistance to flow.

In addition, if the oxygen supply to a conventional respiratory mask fails then leakage around the mask/face seal is generally sufficient to allow the patient to breathe atmospheric air. However, relatively recently, respiratory masks have been developed with a much improved seal between the mask body and the patient's face, as disclosed in published UK patent application GB 2412594. Such devices offer considerable benefits, but the quality of the seal between mask and face does raise a theoretical possibility that if the oxygen supply were to fail then there would be a risk of increased rebreathing for the patient.

An alternative respiratory mask is disclosed in US 5465712.

There has now been devised an improved respiratory mask and improved respiratory gas delivery apparatus that overcome or substantially mitigate the above-mentioned and/or other disadvantages associated with the prior art.

According to a first aspect of the invention, there is provided a respiratory mask comprising a mask body defining a cavity and being adapted to fit about the mouth and/or nose of the patient such that a respiratory gas can be inhaled by the patient from the cavity, a plurality of separate ports for enabling passage of gas into, and out of, the cavity of the mask body, and a valve assembly including a continuous valve membrane having a deformable inhalation valve member that controls flow of gas through an inhalation port and an independently deformable exhalation valve member that controls the flow of gas through an exhalation port, characterised in that the inhalation port includes a valve seat on an interior surface of the mask body, the exhalation port includes a valve seat on an exterior surface of the mask body, and the valve seats of the inhalation and exhalation ports are orientated in substantially the same plane.

The respiratory mask according to this aspect of the invention is advantageous principally because only a single continuous valve membrane is required to control the flow of gas through a plurality of ports, and hence manufacturing costs are significantly reduced.

The inhalation and exhalation valve members may be arranged within substantially the same plane, in a rest configuration of the continuous valve membrane, in which the inhalation and exhalation valve members engage the valve seats of the inhalation and exhalation ports, respectively.

The inhalation and exhalation ports may each include one or more apertures in the mask body through which gas is able to enter and/or exit the cavity of the mask body, during use, the inhalation and exhalation ports being formed integrally with the mask body, such that the mask body including the inhalation and exhalation ports is defined by a single component.

The mask body including the inhalation and exhalation ports may be formed as a single component from plastics material in an injection moulding process.

The valve assembly preferably comprises a single continuous valve membrane. The valve membrane is preferably formed of a plastics material, and may be formed by any suitable manufacturing process, such as by press cutting a sheet of plastics material.

The plurality of ports preferably each include one or more apertures in the mask body through which gas is able to enter and/or exit the cavity of the mask body during use. The plurality of ports are preferably formed integrally with the mask body, such that the mask body including the plurality of ports is defined by a single component. In particularly preferred embodiments, the mask body including the plurality of ports is formed as a single component from plastics material in an injection moulding process.

The valve members are preferably each deformable between a rest configuration in which the valve member occludes the one or more apertures of the corresponding port, and an operative configuration in which that port is at least partially exposed and the passage of gas therethrough is enabled.

The respiratory gas will typically be a mixture of gases, but oxygen will usually constitute a major proportion of that mixture.

The present invention is particularly advantageous for respiratory masks that require both inhalation and exhalation ports, such as so-called high concentration respiratory masks. In this case, the first port is preferably an inhalation port for enabling the supply of gas to the cavity of the mask body, and the second port is preferably an exhalation port for enabling the escape of gas from the cavity of the mask body. In this case, the first valve member is preferably an inhalation member that controls flow of gas through the inhalation port, and the second valve member is preferably an exhalation member that controls the flow of gas through the exhalation port.

The inhalation and exhalation members of the valve membrane are each deformable between inhalation and exhalation configurations, which determine the rate of flow through the inhalation and exhalation ports during a patient's inspiration and expiration phases.

The inhalation member of the valve membrane preferably enables a greater flow of respiratory gas through the inhalation port in the inhalation configuration than in the exhalation configuration. In the case of a non-rebreathing respiratory mask, the inhalation member of the valve membrane preferably acts as a one-way valve by preventing flow of exhaled gas through the inhalation port in the exhalation configuration. However, in order to allow a limited amount of rebreathing, the inhalation member of the valve membrane may enable at least partial flow of exhaled gas through the inhalation port in the exhalation configuration, which may conveniently be achieved by provision of one or more apertures in the inhalation member of the valve membrane. The inhalation member of the valve membrane is preferably deformable between inhalation and exhalation configurations by a change in the differential between the pressure of the gas within the cavity of the mask body and the gas supply pressure.

In presently preferred embodiments, the inhalation member is situated alongside an internal surface of the mask body, and has a rest configuration corresponding to the exhalation configuration in which the inhalation member occludes the inhalation port. The valve membrane is preferably mounted such that a reduction in the pressure within the cavity of the mask body relative to the gas supply pressure, during inspiration by the patient, causes the inhalation member to deform away from, and hence at least partially expose, the inhalation port. This configuration is the inhalation configuration in which flow of gas from the respiratory gas supply conduit into the cavity of the mask body is enabled.

The exhalation member of the membrane preferably enables a greater flow of gas through the exhalation port in the exhalation configuration than in the inhalation configuration. In the case of high concentration respiratory masks, the exhalation member of the membrane preferably acts as a one-way valve by preventing flow of gas through the exhalation port in the inhalation configuration. The exhalation member of the valve membrane is preferably deformable between inhalation and exhalation configurations by a change in the differential between the pressure of the gas within the cavity of the mask body and the surrounding atmospheric pressure.

In presently preferred embodiments, the exhalation member is situated alongside an external surface of the mask body, and has a rest configuration corresponding to the inhalation configuration in which the exhalation member occludes the exhalation port. The valve membrane is preferably mounted such that an increase in the pressure within the cavity of the mask body relative to the pressure of the surrounding atmosphere, during expiration by the patient, causes the exhalation member to deform away from, and hence at least partially expose, the exhalation port. This configuration is the exhalation configuration in which flow of gas from the cavity of the mask body into the surrounding atmosphere is enabled.

The valve membrane preferably therefore has a rest configuration in which the inhalation member occludes the one or more apertures of the inhalation port on the interior surface of the mask body, and the exhalation member occludes the one or more apertures of the exhalation port on the exterior surface of the mask body. The valve membrane is preferably sufficiently resilient that, where there is no pressure differential across the valve membrane, it will revert to its rest configuration. Clearly, the degree of resilience of the valve membrane will vary the pressure differential required to deform the inhalation and exhalation members between their inhalation and exhalation configurations. This resilience can be selected by choosing appropriate dimensions, such as the thickness, of the valve membrane.

The valve membrane is preferably fastened to the mask body at the periphery of the inhalation and exhalation members, such that the inhalation and exhalation members are able to pivot about one or more fastenings on deformation. Most preferably, the valve membrane is fastened to the mask body between the inhalation and exhalation members. This arrangement enables inhalation and exhalation ports having a larger cross-sectional area than for conventional respiratory masks, thereby reducing the resistance to flow during use and hence the work involved in breathing for the patient.

As discussed above, the inhalation member of the valve membrane is preferably situated alongside an interior surface of the mask body, and the exhalation member of the valve membrane is preferably situated on the exterior surface of the mask body. The mask body is preferably therefore provided with a mounting for the valve membrane such that the valve membrane extends through the wall of the mask body. This mounting preferably comprises a slot through which the valve membrane extends, and a shoulder upon which the membrane rests so as to prevent the passage of gas through the slot during use.

The inhalation port and the exhalation port each include a valve seat upon which the valve membrane is able to rest when occluding the one or more apertures of that port in the rest configuration. Each valve seat is preferably continuous, and one or more of the valve seats may have an operative surface that is raised relative to the surrounding surface of the mask body. In presently preferred embodiments, each valve seat is either generally circular or generally semi-circular in shape.

The inhalation port preferably includes a connector for connecting the inhalation port to a supply of respiratory gas. Where the respiratory mask is adapted for providing a high concentration of the respiratory gas supplied to the inhalation port, the connector is preferably adapted for connecting the inhalation port to both a respiratory gas supply conduit and a reservoir bag.

The exhalation port is preferably located at the base of a recess formed in an exterior surface of mask body. This arrangement protects the exhalation port and exhalation member of the valve membrane from damage and accidental occlusion during use.

The respiratory mask preferably also includes one or more safety ports for enabling the supply of atmospheric gas to the cavity of the mask body in the event of a reduced or failed supply of respiratory gas through the inhalation port. Each safety port preferably comprises one or more apertures in the mask body.

In this case, the valve membrane preferably includes one or more safety members that are each deformable between a rest configuration in which the safety member occludes the one or more apertures of a safety port, and an operative configuration in which the one or more apertures of the safety port are at least partially exposed such that atmospheric air is able to flow into the cavity of the mask body. Each safety port preferably includes a valve seat upon which the valve membrane is able to rest when occluding the one or more apertures of that port in the rest configuration. Each valve seat is preferably continuous, and preferably has an operative surface that is raised relative to the surrounding surface of the mask body.

The one or more safety members of the valve membrane are preferably deformable between the rest and operative configurations by a change in the differential between the pressure of the gas within the cavity of the mask body and the pressure of the surrounding atmosphere. In presently preferred embodiments, the one or more safety members are situated alongside an internal surface of the mask body. The valve membrane is preferably mounted such that a reduction in the pressure within the cavity of the mask body relative to the pressure of the surrounding atmosphere, during inspiration by the patient, causes the one or more safety members to deform away from, and hence at least partially expose, the one or more safety ports.

During normal functioning of the respiratory mask, it will generally be desirable for the safety ports to remain closed. For this reason, the one or more safety members are each preferably biased into the rest configuration with a greater force than that biasing the inhalation member. In presently preferred embodiments, the operative surface of the valve seat of each safety port is inclined such that each safety member is inclined relative to the inhalation and exhalation members in the rest configuration. In this arrangement, the resilience of the valve membrane will bias each safety member into the rest configuration with a greater force than that biasing the inhalation member.

The mask body is preferably arranged so that the cavity accommodates the patient's nose and mouth when fitted to the patient. Most preferably, the mask body comprises a mouth portion and a nose portion, the depth of the cavity defined by the nose portion being greater then the depth of the cavity defined by the mouth portion. The inhalation and exhalation ports are preferably arranged in a wall of the mouth portion of the mask body. However, the exhalation port is preferably arranged closer to the nose portion than the inhalation port so that a reservoir bag may extend from the inhalation port over the chin of a patient without occluding the exhalation port during use. In particular, the exhalation port is preferably arranged adjacent to the end of the nose portion of the mask body, such that the recess within which the exhalation port is situated is at least partially defined by that end of the nose portion.

The respiratory mask preferably includes one or more sealing components extending from the peripheral edge of the mask body and defining a contact surface that contacts the face of the patient during use. The contact surface preferably extends along the entire peripheral edge of the mask body so as to seal the cavity against the face of a patient during use. Each sealing component preferably comprises one or more sealing members that define the contact surface. Each sealing member is preferably formed of a resilient material so that it deforms into conformity with the contours of a patient's face during use. In particular, each sealing member is preferably formed of an elastomeric material.

The one or more sealing components are preferably formed from an elastomeric material, which is most preferably a Styrene-Ethylene-Butylene-Styrene (SEBS)-based thermoplastic elastomer. In this case, the respiratory mask is preferably manufactured using a so-called two-shot injection moulding process. In particular, the mask body is preferably injection moulded as a single component of a relatively rigid material, and the elastomeric material of the respiratory mask is then preferably injection moulded onto the surface of the mask body. The mask body and the elastomeric parts of the respiratory mask are bonded together by this process.

In any case, the mask body is preferably formed as a single component of a relatively rigid material. In particular, the mask body is preferably formed so that it maintains its shape when subjected to normal handling, packaging and storage conditions. The mask body is preferably formed from plastics material in an injection moulding process. Most preferably, the mask body is formed of polypropylene. In addition, in presently preferred embodiments, the mask body is at least partially transparent so that the patient is visible through the mask body during use.

The mask body preferably includes retaining formations, such as a fastener, that act to retain the valve membrane within the valve assembly during use. The retaining formations are preferably formed integrally with the mask body, such that the mask body including the retaining formations is defined by a single component. In particularly preferred embodiments, the mask body including the retaining formations is formed as a single component from plastics material in an injection moulding process.

The respiratory mask preferably therefore comprises a mask body defining a cavity and being adapted to fit about the mouth and/or nose of the patient such that inhalation gas can be inhaled by the patient from the cavity, and a valve assembly including a deformable valve membrane for controlling the flow of gas through one or more ports in the mask body, wherein the mask body is manufactured as a single component including integral retaining formations for retaining the valve membrane within the valve assembly during use.

The respiratory mask according to this aspect of the invention is advantageous because only a mask body and a valve membrane are required to define the valve assembly, and hence there is no need for additional components. The cost of manufacture of the respiratory mask is therefore significantly reduced. In addition, the risk of a small component of the respiratory mask becoming detached and blocking a respiratory conduit, during use, is significantly reduced.

Most preferably, the mask body and the valve assembly of the respiratory mask have a form as discussed above in relation to the first aspect of the invention.

The retaining formations preferably include at least one member for restricting movement of the valve membrane along axes that are generally perpendicular to the membrane, and also at least one member for restricting movement of the valve membrane along axes that are generally parallel to the membrane.

In presently preferred embodiments, the retaining formations comprise a retaining clip that extends from a surface of the mask body, through an opening in the valve membrane, and acts to hold the valve membrane against said surface of the mask body. The retaining clip preferably includes an inner member that extends though the opening in the valve membrane, and an outer member that extends along a surface of the valve membrane that faces away from said surface of the mask body. The valve membrane will generally be engaged with the retaining clip by locating a portion of the valve membrane between the outer member of the retaining clip and an adjacent surface of the mask body. The outer member preferably therefore includes a chamfered leading edge to facilitate such location of the valve membrane. The outer member of the retaining clip preferably therefore acts to restrict movement of the valve membrane along axes that are generally perpendicular to the membrane.

The opening in the valve membrane is preferably formed by providing an island in the valve membrane that is deformable independently of the remainder of the membrane. Most preferably, the island has the form of a flap, which is preferably formed by cutting the membrane along a generally C-shaped line. In this case, it is preferred that as little as possible material is removed from the membrane during the cutting operation. This arrangement is particularly advantageous over conventional methods of forming openings in valve membranes, such as punching, because the risk of debris being present in the respiratory mask is significantly reduced. In this case, the flap will generally be deformed away from the opening by the retaining clip during assembly of the respiratory mask, and will rest upon the outer member of the retaining clip when assembled. In this case, the outer member of the retaining clip preferably includes means for restricting movement of the flap, and hence restricting movement of the valve membrane, in the plane of the valve membrane. Such means may take the form of an upstanding projection that impedes movement of the flap relative to the outer member.

Furthermore, the retaining formations preferably also include one or more location projections, such as posts and/or walls, that extend from a surface of the mask body, and act to restrict movement of the valve membrane along axes that are generally parallel to the membrane. In presently preferred embodiments, the valve membrane includes a waist of reduced width, such that a location projection is situated adjacent to the valve membrane on each side of that waist in the valve assembly. Furthermore, one or more location projections are preferably provided that are situated immediately adjacent to those parts of the valve membrane that are movable, in use, and most preferably a plurality of said projections receive a movable part of the valve membrane therebetween with a close fit. Such location projections preferably restrict of the valve membrane along axes that are generally parallel to the membrane, but do not impede movement of said movable part that is necessary, during use, for controlling the flow of gas through the one or more ports in the mask body.

During assembly of the respiratory mask, the clip is preferably passed through the opening in the valve membrane, and the valve membrane is preferably then engaged with the location projections such that the outer member of the clip lies alongside a surface of the membrane. In addition, the mask body is preferably provided with a mounting for the valve membrane such that the valve membrane extends through the wall of the mask body. This mounting preferably comprises a slot through which the valve membrane extends, and a shoulder upon which the membrane rests so as to prevent the passage of gas through the slot during use. In this case, the respiratory mask preferably includes means for impeding, and most preferably preventing, engagement of the valve membrane with the retaining formations if the valve membrane is incorrectly engaged with the mounting, and hence the valve membrane does not extend through the wall of the mask body. Such means preferably takes the form of a projection on an interior surface of the mask that would impede, and most preferably prevent, engagement of the valve membrane with the retaining formations if the valve membrane is incorrectly engaged with the mounting. In presently preferred embodiments, said projection extends from a surface that would be covered by the valve membrane if the valve membrane does not extend through the wall of the mask body, and hence the valve membrane is incorrectly engaged with the mounting, but would not be covered by the valve membrane if the valve membrane is correctly engaged with the mounting.

As discussed above, the present invention is particularly advantageous for respiratory masks that require both inhalation and exhalation ports, such as so-called high concentration respiratory masks. High concentration respiratory masks typically comprise a reservoir bag charged with respiratory gas, so that sufficient respiratory gas is available to meet peak inspiratory flow demands of the patient. In this case, the inhalation port is preferably adapted to connect with both a respiratory gas supply conduit and a reservoir bag.

According to a further aspect of the invention, there is provided respiratory gas delivery apparatus comprising a respiratory mask according to the invention. In presently preferred embodiments, the respiratory gas delivery apparatus includes a reservoir bag adapted for connection to the inhalation port of the respiratory mask. Furthermore, the respiratory gas delivery apparatus preferably includes a respiratory gas supply conduit that is adapted at one end for connection to a supply of respiratory gas, and at the other end for connection to the inhalation port of the respiratory mask.

Preferred embodiments of the invention will now be described in greater detail, by way of illustration only, with reference to the accompanying drawings, in which
Figure 1 is a perspective view of respiratory gas delivery apparatus according to the invention;
Figure 2 is a first perspective view of a first embodiment of a respiratory mask according to the invention, which forms part of the apparatus shown in Figure 1;
Figure 3 is a second perspective view of the respiratory mask of Figure 2;
Figure 4 is a first perspective view of a valve assembly that forms part of the respiratory mask shown in Figures 2 and 3;
Figure 5 is a second perspective view, partly cut-away, of the valve assembly of Figure 4;
Figure 6 is a perspective view of the valve assembly of Figures 4 and 5, with a valve membrane that forms part of the valve assembly having been removed;
Figure 7 is a perspective view of the valve membrane in isolation;
Figure 8 is a first fragmentary perspective view of a reservoir bag connector attached to the respiratory mask, which both form part of the apparatus according to the invention;
Figure 9 is a second fragmentary perspective view, partly cut-away, of the reservoir bag connector attached to the respiratory mask;
Figure 10 is a third fragmentary perspective view, partly cut-away, of the reservoir bag connector attached to the respiratory mask;
Figure 11 is a cross-sectional view of the reservoir bag connector attached to the respiratory mask;
Figure 12 is a first perspective view of a valve assembly that forms part of a second embodiment of a respiratory mask according to the invention; and
Figure 13 is a cross-sectional view of a part of the valve assembly of Figure 12.

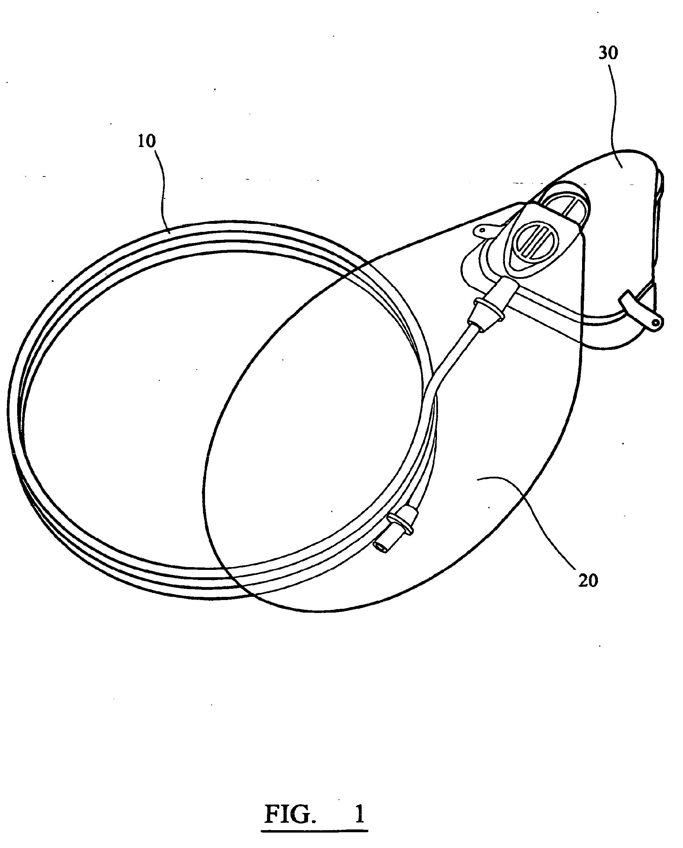

Figure 1 shows respiratory gas delivery apparatus according to the invention comprising a small-bore supply tube 10, a reservoir bag assembly 20, and a respiratory mask 30.

The small-bore supply tube 10 includes a tubular connector (only partially visible in Figure 1) at each end, so that it is adapted for connection to a supply of respiratory gas at one end, and to the reservoir bag assembly 20 at the other end.

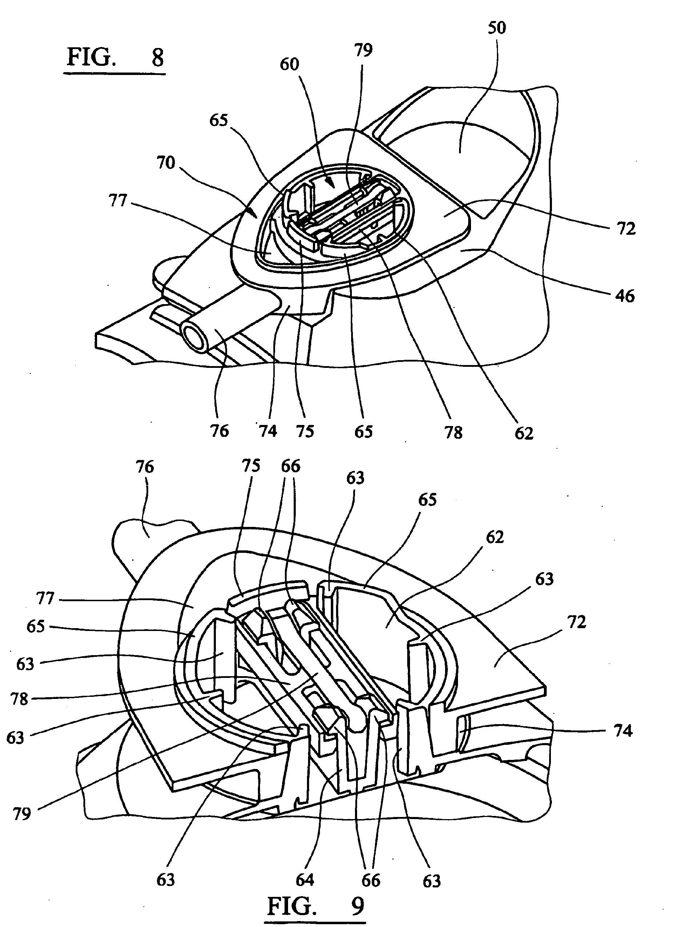

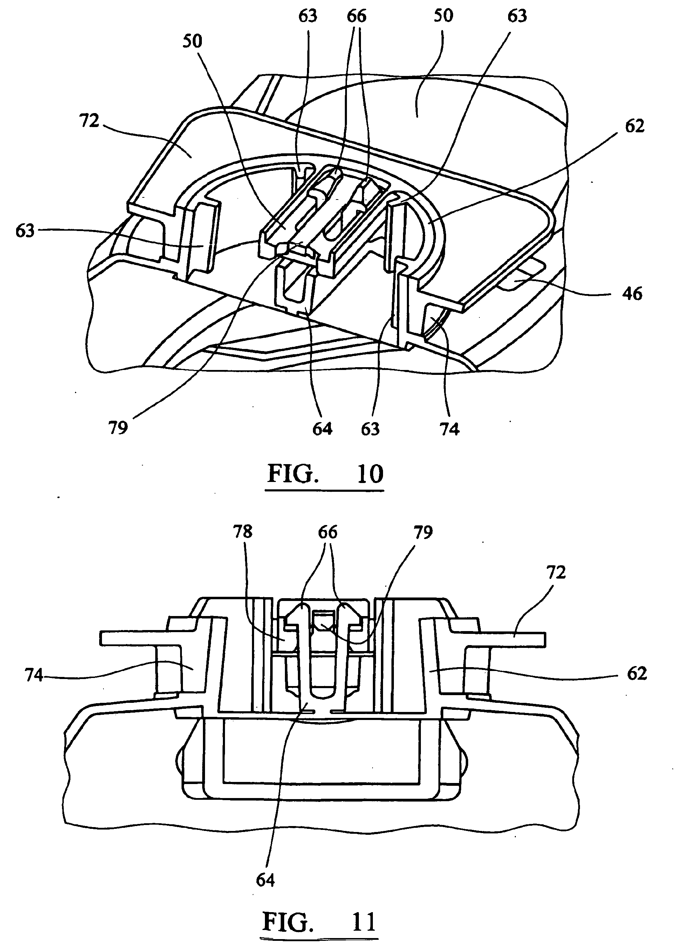

The reservoir bag assembly 20 comprises a flexible bag that is adapted to contain a reservoir of respiratory gas for inhalation by a patient. The flexible bag is therefore connected at one end to both the supply tube 10 and the respiratory mask 30. In particular, the reservoir bag assembly 20 includes a reservoir bag connector 70 for connecting the flexible bag to both the supply tube 10 and the respiratory mask 30. The reservoir bag connector 70 is described in more detail below in relation to Figures 8 to 11.

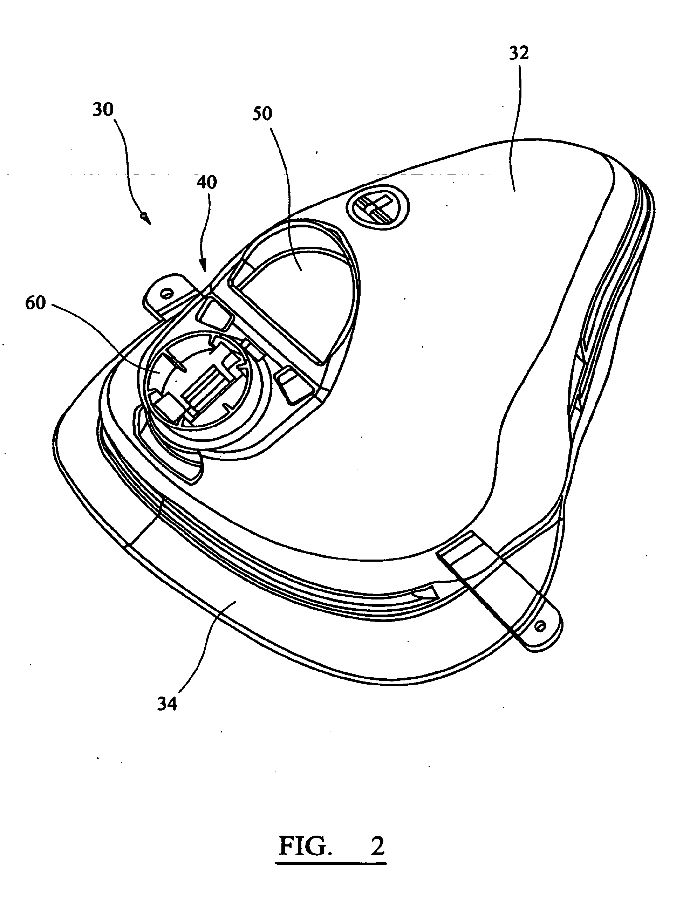

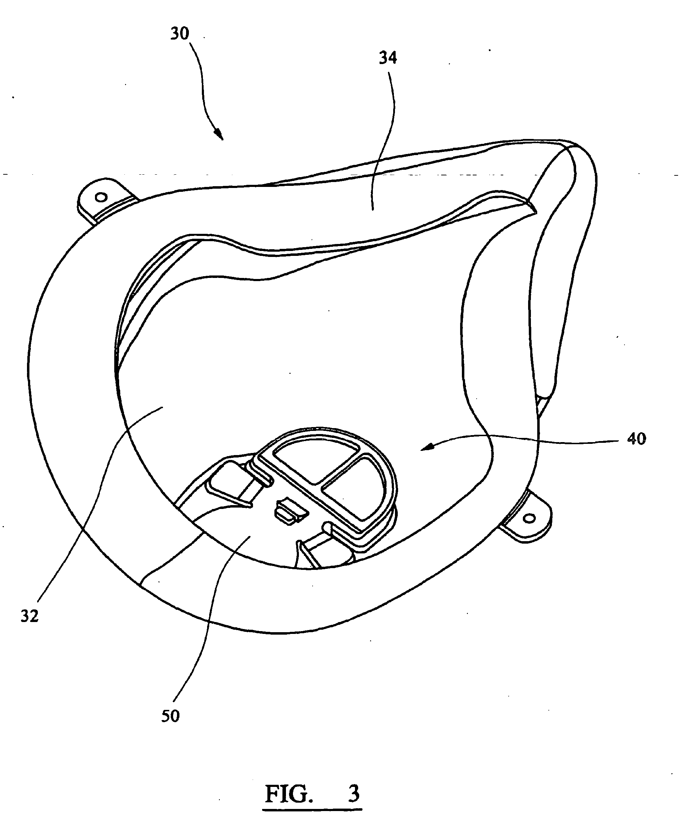

A first embodiment of the respiratory mask 30 is shown more clearly in Figures 2 and 3, and comprises a mask body 32, formed from a suitably strong and relatively rigid plastics material, such as polypropylene, and a sealing flange 34 formed from a suitable plastics material, such as a Styrene-Ethylene-Butylene-Styrene (SEBS)-based thermoplastic elastomer. The respiratory mask 30 is manufactured using a so-called two-shot injection moulding process. In particular, the mask body 32 is firstly injection moulded as a single component, and the sealing flange 34 is then injection moulded onto the surface of the mask body 32. Furthermore, in this embodiment of the invention, the sealing flange 34 has a configuration as described in published UK patent application GB 2412594.

The mask body 32 defines a cavity from which the respiratory gas is delivered to a patient, and comprises a mouth portion and a nose portion. At the mouth portion of the mask body 32, the respiratory mask 30 includes a valve assembly 40 that controls the passage of gases into and out of the cavity defined by the mask body 32.

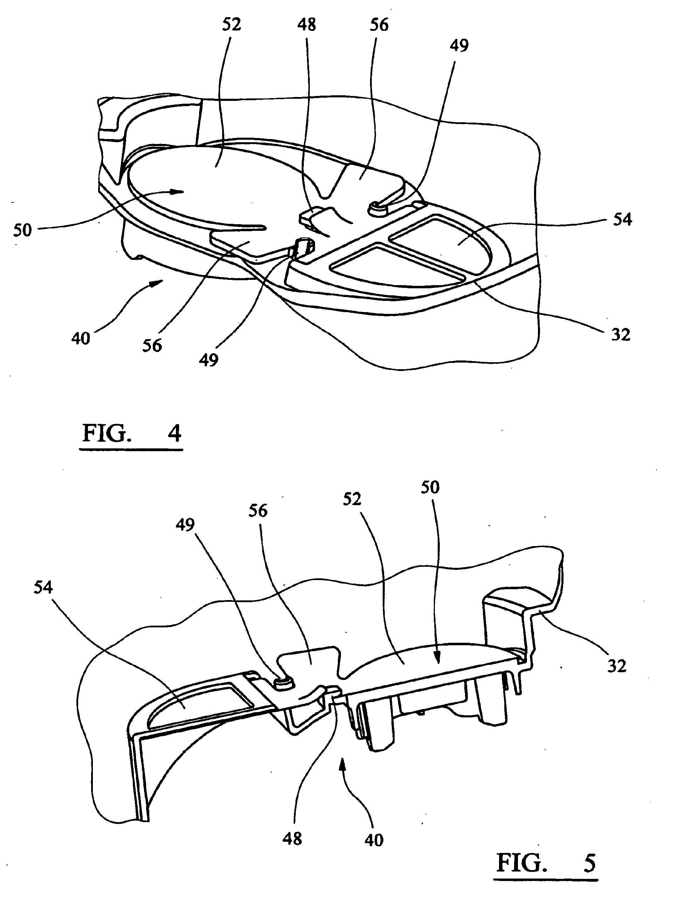

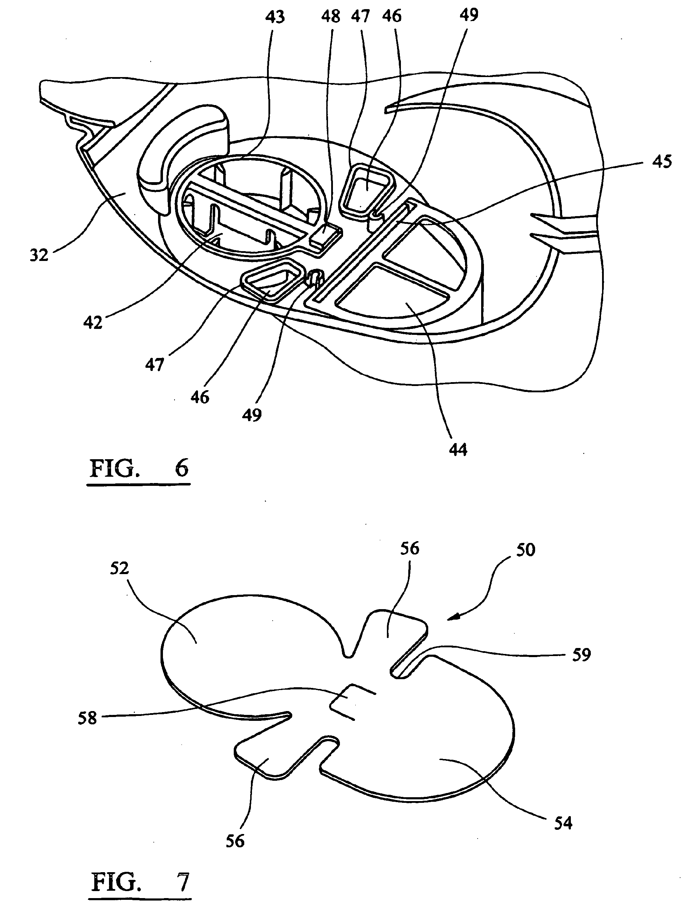

The valve assembly 40 is shown in more detail in Figures 4 and 5, and comprises a valve membrane 50 and valve formations formed integrally with the mask body 32. The valve formations and the valve membrane 50 are shown in isolation in Figures 6 and 7, respectively.

Referring now principally to Figure 6, the valve formations comprise an inhalation port 42, an exhalation port 44, a pair of safety ports 46, a retaining clip 48, and a pair of location posts 49.

The inhalation port 42 comprises a pair of semi-circular apertures in the wall of the mask body 32, and a raised valve seat 43 that surrounds the pair of apertures on the interior surface of the mask body 32. The raised valve seat 43 is circular in shape and has a flat operative surface. As shown more clearly in Figure 2, the inhalation port 42 includes a generally cylindrical connector 60 on the external surface of the mask body 32. The connector 60 is adapted to connect the inhalation port 42 to the reservoir bag assembly 20, as described in more detail below in relation to Figures 8 to 11.

The exhalation port 44 is situated within a generally semi-circular recess in the exterior surface of the mask body 32, and comprises a pair of apertures formed in the base of that recess. A mount 45 for the valve membrane 50 is also provided, which comprises a slot formed in the wall of the mask body 32 such that the membrane 50 extends through the wall of the mask body 32, and a shoulder upon which the membrane 50 rests so as to prevent the passage of gas through the slot during use. The mount 45 is arranged adjacent, and parallel, to the straight edge of the semi-circular recess. In addition, the pair of apertures of the exhalation port 44 are surrounded by a continuous valve seat defined by an external surface of the mask body 32 at the base of the semi-circular recess.

The valve seats of the inhalation and exhalation ports 42,44 are orientated in substantially the same plane, and the mount 45, retaining clip 48 and location posts 49 together act to retain the membrane 50 within the valve assembly during use.

The inhalation and exhalation ports 42,44 are arranged along a central axis of the mask body 32. On each side of this central axis, between the inhalation and exhalation ports 42,44, a safety port 46 is provided. Each safety port 46 comprises a generally trapezoidal aperture in the mask body 32 surrounded by a continuous valve seat 47 that is inclined slightly relative to the plane of the inhalation and exhalation ports 42,44. In particular, the valve seats 47 of the safety ports 46 gradually increase in height as the valve seats 47 extend away from the central axis of the mask body 32.

The retaining clip 48 is L-shaped, with an inner member that projects perpendicularly from the interior surface of the mask body 32 between the safety ports 46, and an outer member that is orientated parallel to the adjacent surface of the mask body 32 and extends towards the inhalation port 42. The location posts 49 project perpendicularly from the interior surface of the mask body 32, and are each positioned between a safety port 46 and the mount 45.

The valve membrane 50 is shown in isolation in Figure 7, and comprises an inhalation member 52, an exhalation member 54, a pair of safety members 56, a central flap 58, and a waist 59 of reduced width relative to the adjacent parts of the membrane 50. The valve membrane 50 is a flat sheet of plastics material, which is formed by press cutting a larger sheet of plastics material, the flap 58 being formed by cutting the membrane 50 along a generally C-shaped line. The flap 58 is therefore deformable independently of the remainder of the membrane 50.

As shown most clearly in Figures 4 and 5, the retaining clip 48 extends through the opening in the valve membrane 50 that is formed by the flap 58, and the outer member of the retaining clip 48 extends over a surface of the membrane 50 that faces away from the interior surface of the mask body 32. In this configuration, the flap 48 rests upon the upper surface of the retaining clip 48, and the location posts 49 are located immediately adjacent to, and on each side of, the waist 59 of the membrane 50. The location posts 49 act to prevent movement of the membrane 50 along axes parallel to the surface of the membrane 50, and hence maintain engagement between the retaining clip 48 and the membrane 50. The mount 45, retaining clip 48 and location posts 49 therefore cooperate to fasten a centre portion of the membrane 50 to the mask body 32.

The inhalation member 52 of the valve membrane 50 is generally circular in shape, and is adapted to occlude the apertures of the inhalation port 42, and engage the corresponding valve seat 43 on the interior surface of the mask body 32, in a rest configuration.

The safety members 56 of the valve membrane 50 extend from a central portion of the valve membrane 50 that is interposed between the inhalation and exhalation members 52,54. The safety members 56 are each generally trapezoidal in shape, and are adapted to occlude the apertures of the safety ports 46, and engage the corresponding valve seats 47 on the interior surface of the mask body 32, in a rest configuration.

The exhalation member 54 of the valve membrane 50 is generally semicircular in shape. In the valve assembly 40, the valve membrane 50 extends over the shoulder, and through the opening, of the mount 45, and projects across the exterior surface of the mask body 32 within the semi-circular recess. The exhalation member 54 is therefore adapted to occlude the apertures of the exhalation port 44, and engage the corresponding valve seat on the exterior of the mask body 32, in a rest configuration.

At rest, the inhalation and exhalation members 52,54 of the valve membrane 50 are arranged within the same plane and engage the parallel valve seats of the inhalation and exhalation ports 42,44, respectively. However, the safety members 56 rest upon the inclined valve seats 47 surrounding the apertures of the safety ports 46, and are therefore inclined relative to the remainder of the valve membrane 50.

The inhalation member 52 of the valve membrane 50 is arranged such that a reduction in the pressure within the cavity of the mask body 32 relative to the pressure within the connector 60, during inspiration by the patient, causes the inhalation member 52 of the valve membrane 50 to pivot about the retaining clip 48 away from the valve seat 43 of the inhalation port 42, so that respiratory gas is drawn into the cavity of the mask body 32 through the apertures of the inhalation port 42. The inhalation member 52 of the valve membrane 50 is also arranged such that an increase in the pressure within the cavity of the mask body 32 relative to the pressure within the connector 60, during expiration by a patient, causes the inhalation member 52 of the valve membrane 50 to pivot about the retaining clip 48 into engagement with the valve seat 43 of the inhalation port 42, so that exhaled gases are not able to escape from the cavity of the mask body 32 through the apertures of the inhalation port 42. In the event that the pressure within the cavity of the mask body 32 is less than the pressure within the connector 60, during expiration by the patient, respiratory gas will enter the cavity of the mask body 32, but exhaled gases will not flow through the inhalation port 42 due to the adverse pressure gradient.

The exhalation member 54 of the valve membrane 50 is arranged such that an increase in the pressure within the cavity of the mask body 32 relative to atmospheric pressure, during expiration by a patient, causes the exhalation member 54 of the valve membrane 50 to pivot about the shoulder of the mount 45 away from the valve seat of the exhalation port 44, so that exhaled gases are able to escape into the atmosphere from the cavity of the mask body 32, through the apertures of the exhalation port 44. The exhalation member 54 of the valve membrane 50 is also arranged such that a reduction in the pressure within the cavity of the mask body 32 relative to atmospheric pressure, during inspiration by a patient, causes the exhalation member 54 of the valve membrane 50 to pivot about the shoulder of the mount 45 into engagement with the valve seat of the exhalation port 44, so that respiratory gas is unable to escape into the atmosphere from the cavity of the mask body 32, through the apertures of the exhalation port 42.

Since the safety members 56 are inclined relative to the remainder of the valve membrane 50, the resilient nature of the valve membrane 50 will resist any deformation of the safety members 56 away from the inclined valve seats 47 with a greater force than that resisting deformation of the inhalation member 52 during inspiration by the patient. The safety members 56 are therefore arranged so that, during normal operation, they will remain in engagement with the valve seats 47 of the safety ports 46, such that atmospheric air is not drawn into the cavity of the mask body 32 during inspiration by the patient. However, in the event that the oxygen supply fails, the pressure within the cavity of the mask body 32 will reduce relative to atmospheric pressure until the reduction in pressure is sufficient to cause deformation of the safety members 56 of the valve membrane 50 away from the valve seats 47 of the safety ports 46, so that gas is drawn from the atmosphere into the cavity of the mask body 32 through the apertures of the safety ports 46.

Turning now to Figures 8 to 11, which each show the respiratory mask connector 60 and the reservoir bag connector 70 in a locked configuration, with the flexible bag omitted for clarity.

The respiratory mask connector 60 comprises a tubular member 62 that projects from the exterior surface of the mask body 32 about the periphery of the inhalation port 42, and a cross-member 64 that extends across a diameter of the tubular member 62, immediately adjacent to the apertures of the inhalation port 42, along a central axis of the mask body 32.

The tubular member 62 includes two recesses in its outer end that accommodate part of the reservoir bag connector 70 in the locked configuration, as described in more detail below, and also six longitudinal strengthening ribs 63 on its interior surface.

The cross-member 64 has a U-shaped cross-section with its arms extending away from the apertures of the inhalation port 42, and being accommodated within the tubular member 62. The arms of the cross-member 64 are generally about one half of the height of the tubular member 62, save for an extended portion at each end of each arm of the cross-member 64. These extended portions define a pair of adjacent locking arms 66 at each end of the cross-member 64.

Each locking arm 66 includes an enlarged head that projects generally perpendicularly away from the adjacent locking arm 66, such that each locking arm 66 includes a surface that faces an aperture of the inhalation port 42. The enlarged head of each locking arm 66 has a tapered and rounded end.

The reservoir bag connector 70 comprises a flange 72 that is heat-bonded or affixed, with glue for example, to an exterior surface of the flexible bag that surrounds an aperture at one end of the flexible bag. In the locked configuration, as shown in Figures 8-11, the flange 72 extends over, but is separated from, the safety ports 46. The flange 72 therefore protects the safety ports 46 from accidental damage and/or occlusion during use.

The reservoir bag connector 70 further comprises a tubular supply connector 76 that extends along a longitudinal axis of the connector 70 towards the chin of the patient when fitted. The supply connector 76 is adapted for connection with a tubular connector of the small-bore supply tube 10. The reservoir bag connector 70 also includes a fluid passageway that leads between the supply connector 76, and a supply aperture 77 that is within the confines of the flange 72 and hence in communication with the aperture of the flexible bag. In this way, respiratory gas is supplied through the supply tube 10, the supply connector 76 and the supply aperture 77 into the flexible bag of the reservoir bag assembly 20, during use.

The reservoir bag connector 70 also comprises a cylindrical sleeve member 74 that extends from the inner edge of the flange 72, adjacent to the supply aperture 77, and is adapted to receive the tubular member 62 of the respiratory mask connector 60 with a close fit.

When the respiratory mask and reservoir bag connectors 60,70 are connected together in the locked configuration, the majority of the outer end of the tubular member 62 is flush with the outer surface of the flange 72. However, the tubular member 62 includes a pair of projections 65 that are situated at each side of the recess in the tubular member 62 that is adjacent to the supply aperture 77. In addition, the cylindrical sleeve member 74 includes a similar projection 75 that is interposed between the projections 65 of the tubular member 62. These projections 65,75 prevent the flexible bag from collapsing, during use, in such a way that gas is unable to pass between the inhalation port 42 and the flexible bag and/or the supply connector 76.

The reservoir bag connector 70 also includes a cross-member 78 that extends along the longitudinal axis of the connector 70 between the supply aperture 77 and a facing part of the flange 72. The cross-member 78 of the reservoir bag connector 70 is adapted to be received within the recesses in the tubular member 62 of the respiratory mask connector 60 and engage the locking arms 66 of that connector 60.

The cross-member 78 of the reservoir bag connector 70 comprises a pair of parallel L-shaped (in cross-section) members that together define a central opening with a ledge on either side, and a pair of resilient arms 79 that are mounted on a central bridge between the L-shaped members and extend in opposite directions along the longitudinal axis of the central opening.

The cross-member 78 of the reservoir bag connector 70, and the locking arms 66 of the respiratory mask connector 60, are adapted to engage one another as the tubular member 62 of the respiratory mask connector 60 is received within the sleeve member 74 of the reservoir bag connector 70. In particular, the enlarged heads of the locking arms 66 are urged against the underside of the L-shaped members of the cross-member 78 of the reservoir bag connector 70.

The tapered and rounded ends of the locking arms 66 are arranged so that the enlarged heads of each adjacent pair of locking arms 66 are deformed towards each other by this action, such that the enlarged heads of the locking arms 66 are then urged against the underside of the resilient arms 79. This causes the outer ends of the resilient arms 79 to be deformed away from the L-shaped members. When the surfaces of the locking arms 66 that face the inhalation port 42 reach the surfaces of the ledges on each side of the opening in the cross-member 78, the locking arms 66 will be able to return to their rest orientation. This will cause the enlarged heads of the locking arms 66 to engage the ledges of the cross-member 78 so as to attach the respiratory mask and respiratory bag connectors 60,70 together. This will also permit the resilient arms 79 to return to their rest configuration, such that the resilient arms 79 are now interposed between the enlarged heads of each pair of adjacent locking arms 66. The presence of the resilient arms 79 between the enlarged heads of the locking arms 66 prevents inward deformation of the locking arms 66, and hence release of the reservoir bag connector 70. The respiratory mask and respiratory bag connectors 60,70 are therefore locked together in this configuration.

Before use, the respiratory mask and respiratory bag connectors 60,70 are locked together, as discussed above, such that the respiratory mask assembly 20 is in communication with the inhalation port 42 of the respiratory mask 30. The supply tube 10 is then connected at one end to a supply of respiratory gas, and at the other end to the supply connector 76 of the respiratory bag connector 70.

Once the respiratory gas delivery apparatus has been assembled, the mask body 32 is secured to a patient's head such that the patient's nose and mouth are accommodated within the cavity defined by the mask body 32.

Respiratory gas is then continuously supplied to the flexible bag of the respiratory bag assembly 20 through the supply connector 76 and supply aperture of the respiratory bag connector 70. In particular, respiratory gas is supplied to the respiratory bag assembly 20 at a rate appropriate for maintaining a reservoir of respiratory gas within the flexible bag that is sufficient for meeting the patient's peak inspiratory demands, without becoming completely depleted.

During use, inhalation by the patient will reduce the pressure within the cavity of the mask body 32 relative to the pressure within the connector 60. This reduction in pressure will act to deform the inhalation member 52 of the valve membrane 50 away from the valve seat 43 of the inhalation port 42, so that respiratory gas is drawn from the reservoir bag assembly 20 into the cavity of the mask body 32 through the apertures of the inhalation port 42.

Exhalation by the patient will increase the pressure within the cavity of the mask body 32 relative to the pressure within the reservoir bag 20 and also atmospheric pressure. This increase in pressure will act to return the inhalation member 52 of the valve membrane 50 into engagement with the valve seat 43 of the inhalation port 42, such that exhaled gases will not enter the reservoir bag. In addition, this increase in pressure will act to deform the exhalation member 54 of the valve membrane 50 away from the valve seat of the exhalation port 44, so that exhaled gases are able to escape into the atmosphere from the cavity of the mask body 32, through the apertures of the exhalation port 44.

In the event that the supply of respiratory gas fails, or the inhalation port 42 becomes blocked, the pressure within the cavity of the mask body 32 will reduce relative to atmospheric pressure until the reduction in pressure is sufficient to cause deformation of the safety members 56 of the valve membrane 50 away from the valve seats 47 of the safety ports 46, so that gas is drawn from the atmosphere into the cavity of the mask body 32 through the apertures of the safety ports 46.

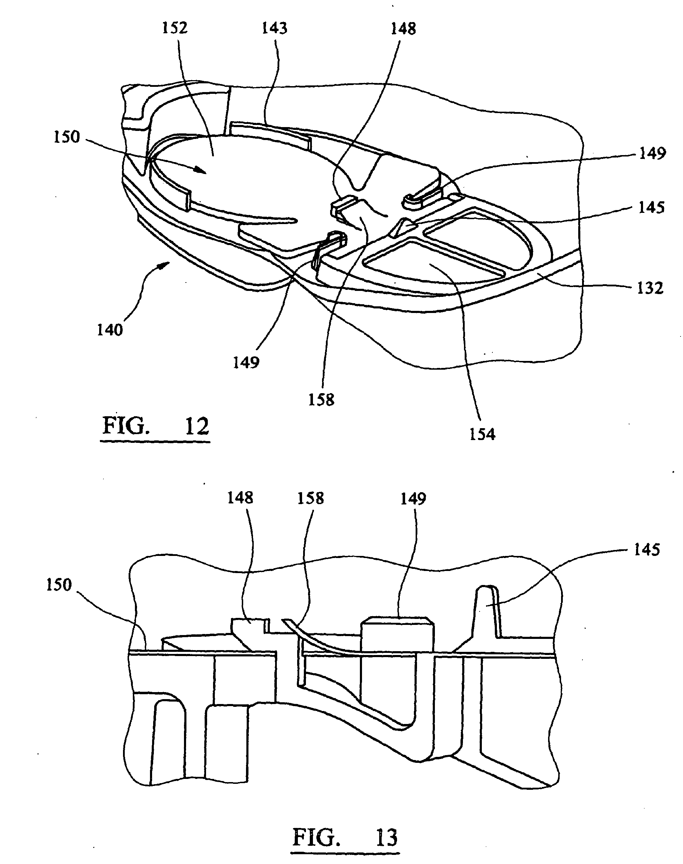

Figures 12 and 13 show a valve assembly 140 that forms part of a second embodiment of a respiratory mask according to the invention. The valve assembly 140 of the second embodiment is identical to the valve assembly 40 of the first embodiment, save for the inclusion of a modified retaining clip 148, modified location posts 149, a pair of retaining skirts 143 and an assembly tab 145.

In particular, the retaining clip 148 includes an additional upstanding projection at the distal end of its outer member, which acts to restrict, and preferably prevent, movement of the valve membrane 150 by providing a barrier for the flap 158 of the membrane. Furthermore, the retaining clip 148 includes a chamfered lower edge under which the membrane 150 is located during engagement with the valve formations, thereby facilitating that engagement.

The location posts 149 of this embodiment are of greater height than the location posts 49 of the first embodiment, and each of the location posts 149 includes an additional retaining wall. The additional retaining walls of the location posts 149 extend alongside the straight edges of the semi-circular exhalation member 154, on each side of the waist of the membrane 150. These features act to improve the engagement between the valve membrane 150 and the valve formations, and also restrict, and preferably prevent, rotation of the valve membrane 150 relative to the valve formations.

The retaining skirts 143 of the second embodiment are upstanding projections that extend about portions of the valve seat of the inhalation valve, such that the inhalation member 152 of the valve membrane 150 is closely received between the retaining skirts 143. The retaining skirts 143 therefore act to restrict, and preferably prevent, movement of the valve membrane 150 relative to the valve formations.

Finally, the assembly tab 145 projects upwardly from an interior surface of the mask body 132 between the apertures and the mount of the exhalation port. Since the valve seat of the exhalation port is defined by an external surface of the mask body 132 surrounding the apertures, the assembly tab 145 will not interfere with correct assembly of the valve membrane 150 within the valve assembly 140. However, if the valve membrane 150 is incorrectly engaged with the valve formations, such that the valve membrane 150 does not extend though the mount to the exterior of the mask body 132, then the assembly tab 145 will prevent engagement of the valve membrane 150 with the location posts 149. This feature therefore prevents an assembled valve configuration in which the exhalation member 154 of the valve membrane 150 is situated on the interior of the mask body 132, and hence in which the exhalation valve would not open during exhalation of the patient.

## Claims

1. A respiratory mask (30) comprising a mask body (32,132) defining a cavity and being adapted to fit about the mouth and/or nose of the patient such that a respiratory gas can be inhaled by the patient from the cavity, a plurality of separate ports (42,44) for enabling passage of gas into, and out of, the cavity of the mask body (32,132), and a valve assembly (40,140) including a continuous valve membrane (50,150) having a deformable inhalation valve member (52,152) that controls flow of gas through an inhalation port (42) and a deformable exhalation valve member (54,154) that controls the flow of gas through an exhalation port (44), **characterised in that** the inhalation valve member (52, 152) and the exhalation valve member (54, 154) are independently deformable, and wherein the inhalation port (42) includes a valve seat on an interior surface of the mask body (32, 132), the exhalation port (44) includes a valve seat on an exterior surface of the mask body (32,132), and the valve seats of the inhalation and exhalation ports (42,44) are orientated in substantially the same plane.

2. A respiratory mask as claimed in Claim 1, wherein the inhalation and exhalation valve members (52,54,152,154) are arranged within substantially the same plane, in a rest configuration of the continuous valve membrane (50,150), in which the inhalation and exhalation valve members (52,54,152,154) engage the valve seats of the inhalation and exhalation ports (42,44), respectively.

3. A respiratory mask as claimed in Claim 1 or Claim 2, wherein the inhalation and exhalation ports (42,44) each include one or more apertures in the mask body (32,132) through which gas is able to enter and/or exit the cavity of the mask body (32,132), during use, the inhalation and exhalation ports (42,44) being formed integrally with the mask body (32,132), such that the mask body (32,132) including the inhalation and exhalation ports (42,44) is defined by a single component.

4. A respiratory mask (30) as claimed in Claim 3, wherein the mask body (32,132) including the inhalation and exhalation ports (42,44) is formed as a single component from plastics material in an injection moulding process.

5. A respiratory mask (30) as claimed in any preceding claim, wherein the valve assembly (40,140) comprises a single continuous valve membrane (50,150).

6. A respiratory mask (30) as claimed in any preceding claim, wherein the inhalation member (52,152) of the valve membrane (50,150) enables at least partial flow of exhaled gas through the inhalation port (42) in an exhalation configuration.

7. A respiratory mask (30) as claimed in Claim 6, wherein one or more apertures are provided in the inhalation member (52,152) of the valve membrane (50,150).

8. A respiratory mask (30) as claimed in any preceding claim, wherein the valve membrane (50,150) has a rest configuration in which the inhalation member (52,152) occludes one or more apertures of the inhalation port (42) on the interior surface of the mask body (32,132), and the exhalation member (54,154) occludes one or more apertures of the exhalation port (44) on the exterior surface of the mask body (32,132).

9. A respiratory mask (30) as claimed in any preceding claim, wherein the valve membrane (50,150) is fastened to the mask body (32,132) between the inhalation and exhalation members (52,54,152,154), such that the inhalation and exhalation members (52,54,152,154) are able to pivot about one or more fastenings (45,48,49,145,148,149) on deformation.

10. A respiratory mask (30) as claimed in any preceding claim, wherein the inhalation member (52,152) of the valve membrane (50,150) is situated alongside
an interior surface of the mask body (32,132), the exhalation member (54,154) of the valve membrane (50,150) is situated on the exterior surface of the mask body (32,132), and the mask body (32,132) is provided with a mounting (45,145) for the valve membrane (50,150) such that the valve membrane (50,150) extends through the wall of the mask body (32,132), the mounting (45,145) comprising a slot through which the valve membrane (50,150) extends, and a shoulder upon which the membrane (50,150) rests so as to prevent the passage of gas through the slot during use.

11. A respiratory mask (30) as claimed in any preceding claim, wherein the exhalation port (44) is located at the base of a recess formed in an exterior surface of mask body (32,132).

12. A respiratory mask (30) as claimed in any preceding claim, wherein the respiratory mask (30) includes one or more safety ports (46) for enabling the supply of atmospheric gas to the cavity of the mask body (32,132) in the event of a reduced or failed supply of respiratory gas through the inhalation port (42).

13. A respiratory mask (30) as claimed in Claim 12, wherein the valve membrane (50,150) includes one or more safety members (56) that are each deformable between a rest configuration in which the safety member (56) occludes one or more apertures of a safety port (46), and an operative configuration in which the one or more apertures of the safety port (46) are at least partially exposed such that atmospheric air is able to flow into the cavity of the mask body (32,132).

14. A respiratory mask (30) as claimed in Claim 13, wherein the one or more safety members (56) are each biased into the rest configuration with a greater force than that biasing the inhalation member (52,152).

15. A respiratory mask (30) as claimed in Claim 14, wherein an operative surface of a valve seat (47) of each safety port (46) is inclined such that each
safety member (56) is inclined relative to the inhalation and exhalation members (52,54,152,154) in the rest configuration.

16. A respiratory mask (30) as claimed in any preceding claim, wherein the mask body (32,132) includes retaining formations (45,48,49,143,145,148,149) that act to retain the valve membrane (50,150) within the valve assembly (40,140) during use.

17. A respiratory mask (30) as claimed in Claim 16, wherein the retaining formations (45,48,49,143,145,148,149) are formed integrally with the mask body (32,132), such that the mask body (32,132) including the retaining formations (45,48,49,143,145,148,149) is defined by a single component.

18. A respiratory mask (30) as claimed in Claim 17, wherein the mask body (32,132) including the retaining formations (45,48,49,143,145,148,149) is formed as a single component from plastics material in an injection moulding process.

19. A respiratory mask (30) as claimed in Claim 18, wherein the retaining formations (45,48,49,143,145,148,149) include at least one member for restricting movement of the valve membrane (50,150) along axes that are generally perpendicular to the membrane (50,150), and at least one member for restricting movement of the valve membrane (50,150) along axes that are generally parallel to the membrane (50,150).

20. A respiratory mask (30) as claimed in Claim 18 or Claim 19, wherein the retaining formations (45,48,49,143,145,148,149) comprise a retaining clip (48,148) that extends from a surface of the mask body (32,132), through an opening in the valve membrane (50,150), and acts to hold the valve membrane (50,150) against said surface of the mask body (32,132).

21. A respiratory mask (30) as claimed in Claim 20, wherein the opening in the valve membrane (50,150) is formed by providing an island (58,158) in the valve membrane (50,150) that is deformable independently of the remainder of the membrane (50,150).

22. A respiratory mask (30) as claimed in Claim 21, wherein the island (58,158) has the form of a flap, which is formed by cutting the membrane (50,150) along a generally C-shaped line.

23. A respiratory mask (30) as claimed in any one of Claims 18 to 22, wherein the retaining formations (45,48,49,143,145,148,149) include one or more location projections (49,143,149) that extend from a surface of the mask body (32,132), and act to restrict movement of the valve membrane (50,150) along axes that are generally parallel to the membrane (50,150).

24. A respiratory mask (30) as claimed in Claim 23, wherein the valve membrane (50,150) includes a waist (59) of reduced width, such that a location projection (49,149) is situated adjacent to the valve membrane (50,150) on each side of that waist (59) in the valve assembly (40,140).

25. A respiratory mask (30) as claimed in any one of Claims 18 to 24, wherein the mask body (32,132) is provided with a mounting (45,145) for the valve membrane (50,150) such that the valve membrane (50,150) extends through the wall of the mask body (32,132), the mounting (45,145) comprising a slot through which the valve membrane (50,150) extends, and a shoulder upon which the membrane (50,150) rests so as to prevent the passage of gas through the slot during use.

26. A respiratory mask (30) as claimed in Claim 25, wherein the respiratory mask (30) includes means for impeding engagement of the valve membrane (50,150) with the retaining formations (45,48,49,143,145,148,149) if the valve membrane (50,150) is incorrectly engaged with the mounting (45,145), and hence
the valve membrane (50,150) does not extend through the wall of the mask body (32,132).

27. Respiratory gas delivery apparatus comprising a respiratory mask (30) as claimed in any preceding claim.

28. Respiratory gas delivery apparatus as claimed in Claim 27, wherein the respiratory gas delivery apparatus includes a reservoir bag (20) adapted for connection to the inhalation port (42) of the respiratory mask (30).

## Patentansprüche

1. Atemmaske (30), die Folgendes umfasst: einen Maskenkörper (32, 132), der einen Hohlraum definiert und so gestaltet ist, dass er um Mund und/oder Nase des Patienten passt, so dass der Patient ein Atemgas aus dem Hohlraum einatmen kann, mehrere separate Ports (42, 44), durch die Gas in den und aus dem Hohlraum des Maskenkörpers (32, 132) strömen kann, und eine Ventilbaugruppe (40, 140) mit einer kontinuierlichen Ventilmembran (50, 150), die ein verformbares Einatmungsventilelement (52, 152) aufweist, das den Durchfluss von Gas durch einen Einatmungsport (42) regelt, und einem verformbaren Ausatmungsventilelement (54, 154), das den Durchfluss von Gas durch einen Ausatmungsport (44) regelt, **dadurch gekennzeichnet, dass** das Einatmungsventilelement (52, 152) und das Ausatmungsventilelement (54, 154) unabhängig verformbar sind, und wobei der Einatmungsport (42) einen Ventilsitz auf einer Innenfläche des Maskenkörpers (32, 132) aufweist, der Ausatmungsport (44) einen Ventilsitz auf einer Außenfläche des Maskenkörpers (32, 132) aufweist, und die Ventilsitze des Ein- und des Ausatmungsports (42, 44) im Wesentlichen in derselben Ebene ausgerichtet sind.

2. Atemmaske nach Anspruch 1, wobei die Ein- und Ausatmungsventilelemente (52, 54, 152, 154) im Wesentlichen in derselben Ebene in einer Ruhekonfiguration der kontinuierlichen Ventilmembran (50, 150) angeordnet sind, in der die Ein- und Ausatmungsventilelemente (52, 54, 152, 154) jeweils in die Ventilsitze des Ein- und des Ausatmungsports (42, 44) eingreifen.

3. Atemmaske nach Anspruch 1 oder Anspruch 2, wobei der Ein- und der Ausatmungsport (42, 44) jeweils eine oder mehrere Öffnungen im Maskenkörper (32, 132) aufweisen, durch die beim Gebrauch Gas in den Hohlraum des Maskenkörpers (32, 132) ein- und/oder daraus austreten kann, wobei der Ein- und der Ausatmungsport (42, 44) einstückig mit dem Maskenkörper (32, 132) ausgebildet sind, so dass der den Ein- und der Ausatmungsport (42, 44) aufweisende Maskenkörper (32, 132) durch eine einzige Komponente definiert wird.

4. Atemmaske (30) nach Anspruch 3, wobei der die Ein- und Ausatmungsports (42, 44) aufweisende Maskenkörper (32, 132) als einzelne Komponente aus Plastikmaterial in einem Spritzformvorgang ausgebildet ist.

5. Atemmaske (30) nach einem der vorherigen Ansprüche, wobei die Ventilbaugruppe (40, 140) eine einzige kontinuierliche Ventilmembran (50, 150) umfasst.

6. Atemmaske (30) nach einem der vorherigen Ansprüche, wobei das Einatmungselement (52, 152) der Ventilmembran (50, 150) in einer Ausatmungskonfiguration wenigstens einen Teildurchfluss von ausgeatmetem Gas durch den Einatmungsport (42) zulässt.

7. Atemmaske (30) nach Anspruch 6, wobei eine oder mehrere Öffnungen im Einatmungselement (52, 152) der Ventilmembran (50, 150) vorgesehen sind.

8. Atemmaske (30) nach einem der vorherigen Ansprüche, wobei die Ventilmembran (50, 150) eine Ruhekonfiguration hat, in der das Einatmungselement (52, 152) eine oder mehrere Öffnungen des Einatmungsports (42) auf der Innenfläche des Maskenkörpers (32, 132) verschließt und das Ausatmungselement (54, 154) eine oder mehrere Öffnungen des Ausatmungsports (44) auf der Außenfläche des Maskenkörpers (32, 132) verschließt.

9. Atemmaske (30) nach einem der vorherigen Ansprüche, wobei die Ventilmembran (50, 150) am Maskenkörper (32, 132) so zwischen den Ein- und Ausatmungselementen (52, 54, 152, 154) befestigt ist, dass die Ein- und Ausatmungselemente (52, 54, 152, 154) bei Verformung um ein oder mehrere Befestigungsmittel (45, 48, 49, 145, 148, 149) drehen können.

10. Atemmaske (30) nach einem der vorherigen Ansprüche, wobei sich das Einatmungselement (52, 152) der Ventilmembran (50, 150) entlang einer Innenfläche des Maskenkörpers (32, 132) befindet, das Ausatmungselement (54, 154) der Ventilmembran (50, 150) sich auf der Außenfläche des Maskenkörpers (32, 132) befindet und der Maskenkörper (32, 132) mit einer Halterung (45, 145) für die Ventilmembran (50, 150) versehen ist, so dass die Ventilmembran (50, 150) durch die Wand des Maskenkörpers (32, 132) verläuft, wobei die Halterung (45, 145) einen Schlitz, durch den die Ventilmembran (50, 150) verläuft, und einen Ansatz umfasst, auf dem die Membran (50, 150) ruht, um den Durchfluss von Gas durch den Schlitz beim Gebrauch zu verhüten.

11. Atemmaske (30) nach einem der vorherigen Ansprüche, wobei sich der Ausatmungsport (44) an der Basis einer in einer Außenfläche des Maskenkörpers (32, 132) ausgebildeten Aussparung befindet.

12. Atemmaske (30) nach einem der vorherigen Ansprüche, wobei die Atemmaske (30) einen oder mehrere Sicherheitsports (46) aufweist, um die Zufuhr von atmosphärischem Gas zum Hohlraum des Maskenkörpers (32, 132) im Falle einer reduzierten oder ausgefallenen Zufuhr von Atemgas durch den Einatmungsport (42) zu ermöglichen.

13. Atemmaske (30) nach Anspruch 12, wobei die Ventilmembran (50, 150) ein oder mehrere Sicherheitselemente (56) aufweist, die jeweils zwischen einer Ruhekonfiguration, in der das Sicherheitselement (56) eine oder mehrere Öffnungen eines Sicherheitsports (46) verschließt, und einer Betriebskonfiguration verformbar sind, in der die eine oder mehreren Öffnungen des Sicherheitsports (46) wenigstens teilweise exponiert sind, so dass atmosphärische Luft in den Hohlraum des Maskenkörpers (32, 132) strömen kann.

14. Atemmaske (30) nach Anspruch 13, wobei die ein oder mehreren Sicherheitselemente (56) jeweils mit einer Kraft in die Ruhekonfiguration vorgespannt werden, die größer ist als die das Einatmungselement (52, 152) vorspannende Kraft.

15. Atemmaske (30) nach Anspruch 14, wobei eine Wirkfläche eines Ventilsitzes (47) jedes Sicherheitsports (46) so geneigt ist, dass jedes Sicherheitselement (56) relativ zu den Ein- und Ausatmungselementen (52, 54, 152, 154) in der Ruhekonfiguration geneigt ist.

16. Atemmaske (30) nach einem der vorherigen Ansprüche, wobei der Maskenkörper (32, 132) Halteformationen (45, 48, 49, 143, 145, 148, 149) aufweist, deren Funktion es ist, die Ventilmembran (50, 150) beim Gebrauch in der Ventilbaugruppe (40, 140) zu halten.

17. Atemmaske (30) nach Anspruch 16, wobei die Halteformationen (45, 48, 49, 143, 145, 148, 149) einstückig mit dem Maskenkörper (32, 132) ausgebildet sind, so dass der die Halteformationen (45, 48, 49, 143, 145, 148, 149) aufweisende Maskenkörper (32, 132) durch eine einzelne Komponente definiert wird.

18. Atemmaske (30) nach Anspruch 17, wobei der die Halteformationen (45, 48, 49, 143, 145, 148, 149) aufweisende Maskenkörper (32, 132) als eine einzelne Komponente aus Plastikmaterial in einem Spritzformvorgang ausgebildet wird.

19. Atemmaske (30) nach Anspruch 18, wobei die Halteformationen (45, 48, 49, 143, 145, 148, 149) wenigstens ein Element zum Beschränken der Bewegung der Ventilmembran (50, 150) entlang Achsen, die zu der Membran (50, 150) allgemein lotrecht sind, und wenigstens ein Element zum Beschränken der Bewegung der Ventilmembran (50, 150) entlang Achsen aufweisen, die zu der Membran (50, 150) allgemein parallel sind.

20. Atemmaske (30) nach Anspruch 18 oder Anspruch 19, wobei die Halteformationen (45, 48, 49, 143, 145, 148, 149) ein Halteclip (48, 148) umfasst, das von einer Oberfläche des Maskenkörpers (32, 132) durch eine Öffnung in der Ventilmembran (50, 150) verläuft und die Funktion hat, die Ventilmembran (50, 150) gegen die genannte Fläche des Maskenkörpers (32, 132) zu halten.

21. Atemmaske (30) nach Anspruch 20, wobei die Öffnung in der Ventilmembran (50, 150) durch Vorsehen einer Insel (58, 158) in der Ventilmembran (50, 150) gebildet wird, die unabhängig vom Rest der Membran (50, 150) verformbar ist.

22. Atemmaske (30) nach Anspruch 21, wobei die Insel (58, 158) die Form einer Klappe hat, die durch Schneiden der Membran (50, 150) entlang einer allgemein C-förmigen Linie gebildet wird.

23. Atemmaske (30) nach einem der Ansprüche 18 bis 22, wobei die Halteformationen (45, 48, 49, 143, 145, 148, 149) einen oder mehrere Positioniervorsprünge (49, 143, 149) aufweisen, die sich von einer Oberfläche des Maskenkörpers (32, 132) erstrecken und die Funktion haben, die Bewegung der Ventilmembran (50, 150) entlang Achsen zu beschränken, die allgemein parallel zur Membran (50, 150) sind.

24. Atemmaske (30) nach Anspruch 23, wobei die Ventilmembran (50, 150) eine Einbuchtung (59) von verringerter Breite aufweist, so dass sich ein Positioniervorsprung (49, 149) neben der Ventilmembran (50, 150) auf jeder Seite dieser Einbuchtung (59) in der Ventilbaugruppe (40, 140) befindet.

25. Atemmaske (30) nach einem der Ansprüche 18 bis 24, wobei der Maskenkörper (32, 132) mit einer Halterung (45, 145) für die Ventilmembran (50, 150) versehen ist, so dass die Ventilmembran (50, 150) durch die Wand des Maskenkörpers (32, 132) verläuft, wobei die Halterung (45, 145) einen Schlitz, durch den die Ventilmembran (50, 150) verläuft, und einen Ansatz aufweist, auf dem die Membran (50, 150) ruht, um den Durchfluss von Gas durch den Schlitz beim Gebrauch zu verhindern.

26. Atemmaske (30) nach Anspruch 25, wobei die Atemmaske (30) Mittel zum Verhindern des Eingreifens der Ventilmembran (50, 150) in die Halteformationen (45, 48, 49, 143, 145, 148, 149) beinhaltet, wenn die Ventilmembran (50, 150) inkorrekt in die Halterung (45, 145) eingreift und die Ventilmembran (50, 150) somit nicht durch die Wand des Maskenkörpers (32, 132) verläuft.

27. Atemgaszuführungsvorrichtung, die eine Atemmaske (30) nach einem der vorherigen Ansprüche umfasst.

28. Atemgaszuführungsvorrichtung nach Anspruch 27, wobei die Atemgaszuführungsvorrichtung einen Vorratsbeutel (20) für den Anschluss am Einatmungsport (42) der Atemmaske (30) aufweist.

## Revendications

1. Un masque respiratoire (30) comprenant un corps de masque (32, 132) définissant une cavité et étant conçu de façon à s'adapter autour de la bouche et/ou du nez du patient, de sorte qu'un gaz respiratoire puisse être inhalé par le patient à partir de la cavité, une pluralité de ports distincts (42, 44) destinés à permettre le passage de gaz vers l'intérieur de, et vers l'extérieur de, la cavité du corps de masque (32, 132), et un ensemble soupape (40, 140) comprenant une membrane de soupape continue (50, 150) possédant un élément de soupape d'inhalation déformable (52, 152) qui commande un écoulement de gaz au travers d'un port d'inhalation (42) et un élément de soupape d'exhalation déformable (54, 154) qui commande le flux de gaz au travers d'un port d'exhalation (44), **caractérisé en ce que** l'élément de soupape d'inhalation (52, 152) et l'élément de soupape d'exhalation (54, 154) sont déformables indépendamment, et où le port d'inhalation (42) comprend un siège de soupape sur une surface intérieure du corps de masque (32, 132), le port d'exhalation (44) comprend un siège de soupape sur une surface extérieure du corps de masque (32, 132), et les sièges de soupape des ports d'inhalation et d'exhalation (42, 44) sont orientés dans sensiblement le même plan.

2. Masque respiratoire selon la Revendication 1, où les éléments de soupape d'inhalation et d'exhalation (52, 54, 152, 154) sont agencés dans sensiblement le même plan, dans une configuration de repos de la membrane de soupape continue (50, 150), dans lequel les éléments de soupape d'inhalation et d'exhalation (52, 54, 152, 154) entrent en prise avec les sièges de soupape des ports d'inhalation et d'exhalation (42, 44), respectivement.

3. Masque respiratoire selon la Revendication 1 ou 2, où les ports d'inhalation et d'exhalation (42, 44) comprennent chacun une ou plusieurs ouvertures dans le corps de masque (32, 132) au travers desquelles du gaz peut entrer et/ou sortir de la cavité du corps de masque (32, 132), en cours d'utilisation, les ports d'inhalation et d'exhalation (42, 44) étant formés intégralement avec le corps de masque (32, 132), de sorte que le corps de masque (32, 132) y compris les ports d'inhalation et d'exhalation (42, 44) est défini par un composant unique.

4. Masque respiratoire (30) selon la Revendication 3, où le corps de masque (32, 132) y compris les ports d'inhalation et d'exhalation (42, 44) est formé sous la forme d'un composant unique à partir d'un matériau plastique dans un processus de moulage par injection.

5. Masque respiratoire (30) selon l'une quelconque des Revendications précédentes, où l'ensemble soupape (40, 140) comprend une membrane de soupape continue unique (50, 150).

6. Masque respiratoire (30) selon l'une quelconque des Revendications précédentes, où l'élément d'inhalation (52, 152) de la membrane de soupape (50, 150) permet au moins un écoulement partiel de gaz exhalé au travers du port d'inhalation (42) dans la configuration d'exhalation.

7. Masque respiratoire (30) selon la Revendication 6, où une ou plusieurs ouvertures sont pourvues dans l'élément d'inhalation (52, 152) de la membrane de soupape (50, 150).

8. Masque respiratoire (30) selon l'une quelconque des Revendications précédentes, où la membrane de soupape (50, 150) possède une configuration de repos dans laquelle l'élément d'inhalation (52, 152) occlut une ou plusieurs ouvertures du port d'inhalation (42) sur la surface intérieure du corps de masque (32, 132), et l'élément d'exhalation (54, 154) occlut une ou plusieurs ouvertures du port d'exhalation (44) sur la surface extérieure du corps de masque (32, 132).

9. Masque respiratoire (30) selon l'une quelconque des Revendications précédentes, où la membrane de soupape (50, 150) est fixée au corps de masque (32, 132) entre les éléments de soupape d'inhalation et d'exhalation (52, 54, 152, 154), de sorte que les éléments de soupape d'inhalation et d'exhalation (52, 54, 152, 154) puissent pivoter autour d'une ou plusieurs pièces de fixation (45, 48, 49, 145, 148, 149) en cas de déformation.

10. Masque respiratoire (30) selon l'une quelconque des Revendications précédentes, où l'élément d'inhalation (52, 152) de la membrane de soupape (50, 150) est situé le long d'une surface intérieure du corps de masque (32, 132), l'élément d'exhalation (54, 154) de la membrane de soupape (50, 150) est situé sur la surface extérieure du corps de masque (32, 132), et le corps de masque (32, 132) est pourvu d'un élément de montage (45, 145) pour la membrane de soupape (50, 150), de sorte que la membrane de soupape (50, 150) s'étende au travers de la paroi du corps de masque (32, 132), l'élément de montage (45, 145) comprenant une fente au travers de laquelle la membrane de soupape (50, 150) s'étend, et un épaulement sur lequel la membrane (50, 150) repose de façon à empêcher le passage de gaz au travers de la fente en cours d'utilisation.

11. Masque respiratoire (30) selon l'une quelconque des Revendications précédentes, où le port d'exhalation (44) est situé à la base d'un évidement formé dans une surface extérieure du corps de masque (32, 132).

12. Masque respiratoire (30) selon l'une quelconque des Revendications précédentes, où le masque respiratoire (30) comprend un ou plusieurs ports de sécurité (46) destinés à permettre la fourniture de gaz atmosphérique à la cavité du corps de masque (32, 132) dans le cas d'une fourniture réduite ou défaillante de gaz respiratoire au travers du port d'inhalation (42).

13. Masque respiratoire (30) selon la Revendication 12, où la membrane de soupape (50, 150) comprend un ou plusieurs éléments de sécurité (56) qui sont chacun déformables entre une configuration de repos dans laquelle l'élément de sécurité (56) occlut une ou plusieurs ouvertures d'un port de sécurité (46) et une configuration opérationnelle dans laquelle les une ou plusieurs ouvertures du port de sécurité (46) sont au moins partiellement exposées de sorte que de l'air atmosphérique puisse s'écouler dans la cavité du corps de masque (32, 132).

14. Masque respiratoire (30) selon la Revendication 13, où les un ou plusieurs éléments de sécurité (56) sont chacun sollicités vers la configuration de repos avec une force plus grande que celle sollicitant l'élément d'inhalation (52, 152).

15. Masque respiratoire (30) selon la Revendication 14, où une surface opérationnelle d'un siège de soupape (47) de chaque port de sécurité (46) est inclinée de sorte que chaque élément de sécurité (56) soit incliné par rapport aux éléments de soupape d'inhalation et d'exhalation (52, 54, 152, 154) dans la configuration de repos.

16. Masque respiratoire (30) selon l'une quelconque des Revendications précédentes, où le corps de masque (32, 132) comprend des formations de retenue (45, 48, 49, 143, 145, 148, 149) qui agissent de façon à retenir la membrane de soupape (50, 150) à l'intérieur de l'ensemble soupape (40, 140) en cours d'utilisation.

17. Masque respiratoire (30) selon la Revendication 16, où les formations de retenue (45, 48, 49, 143, 145, 148, 149) sont formées intégralement avec le corps de masque (32, 132), de sorte que le corps de masque (32, 132) y compris les formations de retenue (45, 48, 49, 143, 145, 148, 149) soit défini par un composant unique.

18. Masque respiratoire (30) selon la Revendication 17, où le corps de masque (32, 132) y compris les formations de retenue (45, 48, 49, 143, 145, 148, 149) est formé sous la forme d'un composant unique à partir d'un matériau plastique dans un processus de moulage par injection.

19. Masque respiratoire (30) selon la Revendication 18, où les formations de retenue (45, 48, 49, 143, 145, 148, 149) comprennent au moins un élément destiné à restreindre le déplacement de la membrane de soupape (50, 150) le long d'axes qui sont généralement perpendiculaires à la membrane (50, 150), et au moins un élément destiné à restreindre le déplacement de la membrane de soupape (50, 150) le long d'axes qui sont généralement parallèles à la membrane (50, 150).

20. Masque respiratoire (30) selon la Revendication 18 ou 19, où les formations de retenue (45, 48, 49, 143, 145, 148, 149) comprennent une pince de retenue (48, 148) qui s'étend à partir d'une surface du corps de masque (32, 132) au travers d'une ouverture dans la membrane de soupape (50, 150) et qui agit de façon à maintenir la membrane de soupape (50, 150) contre ladite surface du corps de masque (32, 132).

21. Masque respiratoire (30) selon la Revendication 20, où l'ouverture dans la membrane de soupape (50, 150) est formée par la fourniture d'un îlot (58, 158) dans la membrane de soupape (50, 150) qui est déformable indépendamment du reste de la membrane (50, 150).

22. Masque respiratoire (30) selon la Revendication 21, où l'îlot (58, 158) a la forme d'un rabat, qui est formé par un découpage de la membrane (50, 150) le long d'une ligne généralement en forme de C.

23. Masque respiratoire (30) selon l'une quelconque des Revendications 18 à 22, où les formations de retenue (45, 48, 49, 143, 145, 148, 149) comprennent une ou plusieurs projections d'emplacement (49, 143, 149) qui s'étendent à partir d'une surface du corps de masque (32, 132) et agissent de façon à restreindre le déplacement de la membrane de soupape (50, 150) le long d'axes qui sont généralement parallèles à la membrane (50, 150).

24. Masque respiratoire (30) selon la Revendication 23, où la membrane de soupape (50, 150) comprend une cambrure (59) de largeur réduite, de sorte qu'une projection d'emplacement (49, 149) soit située de manière adjacente à la membrane de soupape (50, 150) sur chaque côté de cette cambrure (59) dans l'ensemble soupape (40, 140).

25. Masque respiratoire (30) selon l'une quelconque des Revendications 18 à 24, où le corps de masque (32, 132) est pourvu d'un élément de montage (45, 145) pour la membrane de soupape (50, 150), de sorte que la membrane de soupape (50, 150) s'étende au travers de la paroi du corps de masque (32, 132), l'élément de montage (45, 145) comprenant une fente au travers de laquelle la membrane de soupape (50, 150) s'étend et un épaulement sur lequel la membrane (50, 150) repose de façon à empêcher le passage de gaz au travers de la fente en cours d'utilisation.

26. Masque respiratoire (30) selon la Revendication 25, où le masque respiratoire (30) comprend un moyen d'empêcher la mise en prise de la membrane de soupape (50, 150) avec les formations de retenue (45, 48, 49, 143, 145, 148, 149) si la membrane de soupape (50, 150) est incorrectement en prise avec l'élément de montage (45, 145), et dès lors la membrane de soupape (50, 150) ne s'étend pas au travers de la paroi du corps de masque (32, 132).

27. Appareil de distribution de gaz respiratoire comprenant un masque respiratoire (30) selon l'une quelconque des Revendications précédentes.

28. Appareil de distribution de gaz respiratoire selon la Revendication 27, où l'appareil de distribution de gaz respiratoire comprend un sac réservoir (20) adapté pour un raccordement au port d'inhalation (42) du masque respiratoire (30).
